# EUROPEAN PATENT SPECIFICATION

(11) **EP 4 590 245 B1**
(45) Date of publication and mention of the grant of the patent: **27.05.2026**
(21) Application number: 23793098.7
(22) Date of filing: 18.09.2023
(51) Int. Cl.: A61F 5/00

(54) **DEVICES IMPLANTABLE ACROSS DYNAMIC ANATOMICAL PASSAGES AND ASSOCIATED SYSTEMS AND METHODS**
ÜBER DYNAMISCHE ANATOMISCHE DURCHGÄNGE IMPLANTIERBARE VORRICHTUNGEN SOWIE ZUGEHÖRIGE SYSTEME UND VERFAHREN
DISPOSITIFS IMPLANTABLES À TRAVERS DES PASSAGES ANATOMIQUES DYNAMIQUES ET SYSTÈMES ET MÉTHODES ASSOCIÉS

(30) Priority: 19.09.2022 US 202263407948 P
(43) Date of publication of application: 30.07.2025
(73) Proprietor: Boston Scientific Scimed, Inc., Maple Grove, MN 55311-1566 (US)
(72) Inventor: CALVO, Yeison, San Ramón (CR); FAVREAU, John Thomas, Shrewsbury, Massachusetts 01545 (US)
(74) Representative: Vossius & Partner Patentanwälte Rechtsanwälte mbB
(86) International application number: PCT/US2023/033026
(87) International publication number: WO 2024/064074

(56) References cited:
- US-A1- 2015 196 410
- US-A1- 2021 038 414

## Description

### FIELD

The present disclosure relates generally to the field of implantable medical devices. More particularly, the present disclosure relates to medical devices implantable across or within a dynamic anatomical passage capable of moving and exerting forces on the implantable medical device, and associated systems and methods.

### BACKGROUND

Various medical procedures or treatments involve placing an implantable medical device with respect to (across, through, etc.) an anatomical passage, such as to occlude flow of materials through such passage. For instance, treatment methods for various medical conditions, such as obesity, diabetes, or duodenal ulcers, involve restricting flow of materials through the duodenum or even bypassing the duodenum. In the case of a gastric bypass, a duodenal occlusion or exclusion device may be placed in the pyloric sphincter to inhibit or block passage of materials (fluid, chyme, etc.) from the stomach through the pylorus and into the duodenum. Gastric materials are redirected to a bypass, such as a gastrojejunal bypass formed by an anastomosis between the stomach and a portion of the jejunum. Various challenges to preventing migration of a deployed occlusion device are presented by the natural movements of the body (e.g., the gastrointestinal system) as well as the constant flow of materials against the occlusion device. Peristaltic movement of the pylorus to pass materials therethrough (e.g., distally into the small intestine), generally less frequent reverse peristalsis through the pylorus (proximally into the stomach), as well as the natural tendency of the pylorus to eject materials therein, present particular challenges for placement and retention of pyloric occlusion devices. However, formation of an implantable device to be resistant to migration may impact the ability of the implantable device to withstand repetitive movements or waves of anatomical forces impacting the device. For instance, a medical device implanted with respect to a pylorus typically must withstand regular / continuous peristaltic waves from the antrum of the stomach. Such forces may particularly impact welds of the implantable medical device and/or relatively stiff sections and/or angled surfaces of the implantable medical device designed to resist forces and thereby to retain the device in place against forces tending to cause migration of the device. Constant, continuous, and/or regular impact of forces on the implantable medical device may cause compression or deformation of regions of the device which, if excessive, may cause fatigue and/or failures of the device. Accordingly, there is an ongoing need for solutions to the various challenges faced by medical devices implanted with respect to anatomical passages, particularly dynamic anatomical passages which move periodically or frequently.

US 2021/038414 A1 discloses a gastric diverter and a digestive tract support and a release method thereof. The digestive tract support has undeployed shape and deployed shape, and includes upper support, lower support and connecting member. In the deployed shape, a first opening is provided at top of the upper support, a second opening is provided at bottom thereof, and the lower support is disposed below the upper support; a third opening is provided at top of the lower support, a fourth opening is provided at bottom thereof, and the upper support and the lower support are connected by a plurality of connecting members; the fourth opening of the lower support is connected to a membrane tube, the deployed upper support and lower support both cannot pass through an open gastric pyloric orifice, and the connecting members can pass through the gastric pyloric orifice or be placed at the gastric pyloric orifice.

### SUMMARY

This Summary is provided to introduce, in simplified form, a selection of concepts described in further detail below in the Detailed Description. This Summary is not intended to necessarily identify key features or essential features of the claimed subject matter, nor is it intended as an aid in determining the scope of the claimed subject matter. One of skill in the art will understand that each of the various aspects and features of the present disclosure may advantageously be used separately in some instances, or in combination with other aspects and features of the disclosure in other instances, whether or not described in this Summary.

The present invention is defined by the independent claim. Optional embodiments are defined in the dependent claims.

In some embodiments, the first retention member is formed of a compliant material capable of flexing with radially-inwardly directed anatomical forces applied thereto. In some embodiments, the first retention member is compressible from an initial expanded configuration in response to application of radially-inwardly forces applied thereto, and is formed of a resilient material returning the first retention member to the expanded configuration upon the force subsiding. In some embodiments, the first retention member presents a convex tissue-contacting surface to tissue at the deployment site, and is configured and dimensioned to resist migration from the deployment site.

In some embodiments, the body is formed of a tubular member, and at least the first retention member is formed by inverting an end of the tubular member and extending the free end of the inverted end toward the midsection of the tubular member a sufficient distance to cause the inverted end of the tubular member to resist returning to its initial uninverted tubular configuration. In some embodiments, the tubular member is formed from a plurality of interwoven filaments.

In some embodiments, the first retention member is formed from a bowl-shaped element separate from the body. In some embodiments, the first retention member is movable with respect to the body. In some embodiments, the body is formed from an elastic material allowing the first retention member to be moved away from the second retention member.

In some embodiments, the tissue-contacting surface of the first retention member is convex.

In accordance with various principles of the present disclosure, an implantable medical device, configured to be implanted with respect to a pylorus of a patient, includes a body having a first end, a second end, and a midsection extending therebetween; a gastric retention member extending radially-outwardly from the first end of the body; and a duodenal retention member extending radially-outwardly from the second end of the body. In some embodiments, the gastric retention member has a tissue-contacting surface extending radially outwardly from the first end of the body and toward the midsection of the body to a free edge of the gastric retention member spaced from the first end of the body in a direction toward the midsection of the body a sufficient extent to remain in such configuration when the implantable medical device is implanted with respect to the patient's pylorus with the gastric retention member positioned within the patient's stomach. In some embodiments, the gastric retention member is resiliently compliant to contract from an expanded configuration to a compressed configuration upon application of peristaltic forces thereto from the antrum of the patient's stomach, and to return to the expanded configuration upon passing of a peristaltic wave from the gastric retention member.

In some embodiments, the gastric retention member presents a convex tissue-contacting surface to the antrum of the patient's stomach.

In some embodiments, the implantable medical device is formed from a tubular member having a first end, a second end, and a midsection therebetween; and the gastric retention member is formed by inverting the first end of the tubular member and pulling the free end thereof towards the second end of the tubular member a sufficient distance to remain in the inverted configuration when implanted with respect to the antrum of the patient's stomach and when compressed by a peristaltic wave of the antrum.

In some embodiments, the body and at least the gastric retention member are formed separately and coupled together to allow relative movement therebetween. In some embodiments, the body is formed of an elastic material allowing the gastric retention member to move away from the duodenal retention member.

In accordance with various principles of the present disclosure, a method of at least partially occluding flow of materials through a pylorus of a patient includes deploying a body of an implantable medical device across the pylorus; deploying a gastric retention member of the implantable medical device in the antrum of the patient; and deploying a duodenal retention member of the implantable medical device in the duodenum of the patient. In some aspects, the gastric retention member has a tissue-contacting surface extending radially-outwardly from a first end of the body of the implantable medical device and towards a midsection of the body, and the gastric retention member is formed of a compliant material compressing in response to peristaltic contractions of the antrum and returning to an expanded configuration upon passing of a peristaltic contraction of the antrum. In some aspects, the duodenal retention member extends radially-outwardly from a second end of the body of the implantable medical device.

In some aspects, the implantable medical device is formed from a tubular member having a first end and a second end, with the first end inverted to form the gastric retention member of the implantable medical device, the method comprising placing the inverted first end in contact with the antrum with the free end of the tubular member positioned inwardly of the first end of the tubular member in a direction toward the second end of the tubular member.

In some aspects, the gastric retention member presents a convexly curved tissue-contacting surface, the method comprising placing the concavely-curved tissue-contacting surface in contact with the patient's antrum for contracting in response to peristaltic waves applied thereto.

In some aspects, the gastric retention member extends a sufficient distance from the first end of the body to the midsection of the body to resist inversion causing the gastric retention member to extend away from the midsection of the body.

In some aspects, the implantable medical device is formed from a body separate from the gastric retention member such that upon placement of the implantable medical device with respect to the patient's pylorus, the gastric retention member is capable of movement with respect to the body of the implantable medical device.

These and other features and advantages of the present disclosure, will be readily apparent from the following detailed description, the scope of the claimed invention being set out in the appended claims. While the following disclosure is presented in terms of aspects or embodiments, it should be appreciated that individual aspects can be claimed separately or in combination with aspects and features of that embodiment or any other embodiment.

### BRIEF DESCRIPTION OF THE DRAWINGS

Non-limiting embodiments of the present disclosure are described by way of example with reference to the accompanying drawings, which are schematic and not intended to be drawn to scale. The accompanying drawings are provided for purposes of illustration only, and the dimensions, positions, order, and relative sizes reflected in the figures in the drawings may vary. For example, devices may be enlarged so that detail is discernable, but is intended to be scaled down in relation to, *e.g.,* fit within a working channel of a delivery device such as a catheter or endoscope. In the figures, identical or nearly identical or equivalent elements are typically represented by the same reference characters, and similar elements are typically designated with similar reference numbers differing in increments of 100, with redundant description omitted. For purposes of clarity and simplicity, not every element is labeled in every figure, nor is every element of each embodiment shown where illustration is not necessary to allow those of ordinary skill in the art to understand the disclosure.

The detailed description will be better understood in conjunction with the accompanying drawings, wherein like reference characters represent like elements, as follows:
**FIG. 1** illustrates an elevational view of an example of an embodiment of an implantable medical device formed in accordance with various aspects of the present disclosure and positioned in a schematic representation of a gastrointestinal environment.
**FIG. 2** illustrates an elevational view of an example of an embodiment of an implantable medical device such as illustrated in **FIG. 1** with biological forces schematically illustrated as being applied thereto.
**FIG. 3** illustrates a cross-sectional view along line **III-III** of the example of an embodiment of an implantable medical device illustrated in **FIG. 2****.**
**FIG. 4** illustrates an example of an embodiment of an implantable medical device formed in accordance with various principles of the present disclosure.
**FIG. 5** illustrates an example of an embodiment of an implantable medical device formed in accordance with various principles of the present disclosure.
**FIG. 6** illustrates a cross-sectional view along line **VI-VI** of the example of an embodiment of an implantable medical device illustrated in **FIG. 5****.**

### DETAILED DESCRIPTION

It will be appreciated that the present disclosure is set forth in various levels of detail in this application. In certain instances, details that are not necessary for one of ordinary skill in the art to understand the disclosure, or that render other details difficult to perceive may have been omitted. The terminology used herein is for the purpose of describing particular embodiments only, and is not intended to be limiting beyond the scope of the appended claims. Unless defined otherwise, technical terms used herein are to be understood as commonly understood by one of ordinary skill in the art to which the disclosure belongs. All of the devices and/or methods disclosed and claimed herein can be made and executed without undue experimentation in light of the present disclosure.

As used herein, "proximal" refers to the direction or location closest to the user (medical professional or clinician or technician or operator or physician, etc., such terms being used interchangeably herein without intent to limit, and including automated controller systems or otherwise), etc., such as when using a device (e.g., introducing the device into a patient, or during implantation, positioning, or delivery), and/or closest to a delivery device, and "distal" refers to the direction or location furthest from the user, such as when using the device (e.g., introducing the device into a patient, or during implantation, positioning, or delivery), and/or closest to a delivery device. "Longitudinal" means extending along the longer or larger dimension of an element. A "longitudinal axis" extends along the longitudinal extent of an element, though is not necessarily straight and does not necessarily maintain a fixed configuration if the element flexes or bends, and "axial" generally refers to along the longitudinal axis. However, it will be appreciated that reference to axial or longitudinal movement with respect to the above-described systems or elements thereof need not be strictly limited to axial and/or longitudinal movements along a longitudinal axis or central axis of the referenced elements. "Central" means at least generally bisecting a center point and/or generally equidistant from a periphery or boundary, including regions in the vicinity thereof, and a "central axis" means, with respect to an opening, a line that at least generally bisects a center point of the opening, extending longitudinally along the length of the opening when the opening comprises, for example, a tubular element, a channel, a cavity, or a bore. As used herein, a "lumen" or "channel" or "bore" or "passage" is not limited to a circular cross-section. As used herein, a "free end" of an element is a terminal end at which such element does not extend beyond. It will be appreciated that terms such as at or on or adjacent or along or within may be used interchangeably herein without intent to limit unless otherwise stated, and are intended to indicate a general relative spatial relation rather than a precisely limited location. Finally, reference to "at" a location or site is intended to include at and/or about the vicinity of (e.g., along, adjacent, etc.) such location or site.

The present disclosure relates to implantable medical devices having a body with a first end, a second end, and a midsection therebetween. The implantable medical device may be configured to be expandable from a collapsed or contracted delivery configuration (e.g., facilitating intraluminal / transluminal delivery to a deployment site through body passages without requiring open surgery for delivery of the device) to an expanded deployed configuration. The midsection of the implantable medical device is deployable across or along or at (such terms being used interchangeably herein without intent to limit) a deployment site within a body with a retention member at one or both ends thereof extending radially outward therefrom to retain the implantable medical device in place at the deployment site. It will be appreciated that reference may be made herein to a deployment site, anatomical site, delivery site, implant / implantation site, site of implantation, target site, treatment site, etc., interchangeably and without intent to limit. A non-limiting example of a deployment site referenced herein is an anatomical passage, which may be any passage or lumen within a patient's body, and may be referenced alternatively herein as a body passage, body lumen, anatomical lumen, etc., without intent to limit. It will be appreciated that reference to a body passage includes naturally-existing passages (e.g., the pylorus) as well as medically-created passages (e.g., a passage created with the use of a medical instrument, such as between a stomach and jejunum) or otherwise. One or both ends of the implantable medical device have a retention member configured to engage tissue walls surrounding ends of the anatomical passage to hold the implantable medical device in place and to resist migration of the implantable medical device from its deployment site.

At least a portion of various anatomical deployment sites in which an implantable medical device may be deployed may be subjected to various forces, movements, etc., which may put pressure or stress on the implantable medical device. Over extended periods of time, an implantable medical device subjected to forces at its deployment site (e.g., from dynamic anatomical tissue at the deployment site) may exhibit degradation, fatigue, or even points of failure. For instance, some implantable medical devices are formed of woven filaments welded at the free ends of the device to finish and hold the woven configuration in place without unravelling. Repeated and prolonged pressure on such devices may ultimately affect the welds at the free ends, potentially causing at least partial unravelling of the woven device.

In accordance with various principles of the present disclosure, at least one retention member of an implantable medical device is compliant and configured to flex in response to a force applied thereto, rather than to resist the force, while also still holding the implantable medical device in place at the deployment site. It will be appreciated that the term flex (including other grammatical forms thereof) is used herein for the sake of convenience and broadly and generally covers a level of compliance allowing the device to move with or to "ride" with and/or absorb forces in contrast with resisting forces, various such terms thus being used interchangeably herein without intent to limit. Without being bound by theory, flexing in response to forces such as naturally-occurring anatomical forces (e.g., peristaltic waves in the case of placement in the gastrointestinal tract), rather than resisting forces, may lead to less stress and thus less wear and tear and potential degradation.

More particularly, various anatomical deployment sites may subject a device implanted at such site to forces such as radially-inwardly directed forces. In accordance with various principles of the present disclosure, at least one retention member of an implantable medical device is sufficiently compliant to move with or to "ride" with anatomical forces applied thereto and to be compressed by such forces, rather than resist such forces. The at least one retention member is sized, shaped, configured, and/or dimensioned to resist migration from the deployment site even if compressed.

For the sake of simplicity, some descriptions herein are with reference to "a" retention member rather than to both retention members. It should be appreciated that such references are not intended to be limited to a single retention member, and may apply to both retention members of an implantable medical device formed in accordance with various principles of the present disclosure.

In some aspects, at least one retention member of an implantable medical device formed in accordance with various principles of the present disclosure presents an extended tissue-contacting surface for contacting tissue at the deployment site of the device. For instance, in some embodiments, a midsection of an implantable medical device is deployed across an anatomical passage, with a retention member expanded outside the anatomical passage and in contact with tissue surrounding an opening into the anatomical passage. In accordance with various principles of the present disclosure, the tissue-contacting surface of the retention member extends from a generally central region thereof (optionally adjacent the longitudinal axis of the implantable medical device and/or the body thereof) radially-outwardly from the longitudinal axis and longitudinally away from the end of the implantable medical device from which the retention member extends and towards the midsection of the implantable medical device and the opposite end of the implantable medical device. In such embodiments, the retention member may present a convex surface to the tissue surrounding the opening to the anatomical passage to anchor the implantable medical device in place at the deployment site. Even more particularly, in accordance with various principles of the present disclosure, a retention member of an implantable medical device may have a bowl-shape, "mushroom," or "umbrella" configuration, presenting an extended convex tissue-contacting surface for engaging and anchoring with respect to tissue at the deployment site. The inner surface of the retention member wall may be concave. The configuration of a retention member with at least a convex outer, tissue-contacting surface, and optionally also a concave inner surface (radially-inwardly directed, on the opposite side of the retention member wall), may result in a shape / configuration resistant to inversion. In such a bowl-shaped configuration, the retention member may be more resistant to migration from the deployment site than in an inverted configuration. As may be appreciated, the radially-outward extent of the tissue-contacting surface from the midsection and/or longitudinal axis of the implantable medical device to a free end or edge of the retention member (e.g., a terminal end or edge of the surface or structure in general) is sufficiently long, and provides sufficient surface area for engagement with tissue at the deployment site to retain the implantable medical device in place and to resist migration from the deployment site. For purposes of the present disclosure, terms such as anchor, affix, associate, deploy on, engage hold, retain, secure, etc. (and other grammatical forms thereof), may be used interchangeably herein without intent to limit.

Moreover, it will be appreciated that the extension of the retention member radially from the longitudinal axis of the implantable medical device may also contribute to the ability of the retention member to retain the implantable medical device in place with respect to a deployment site such as an anatomical passage. In order for the retention member to ride with anatomical forces in accordance with various principles of the present disclosure, while also retaining the implantable medical device in place at the deployment site, the retention member should have a radial extent (transverse to the longitudinal axis of the midsection and/or the device as a whole) sufficiently large to retain the implantable medical device in place at the deployment site. For instance, in some aspects, a retention member formed in accordance with various principles of the present disclosure to flex in response to anatomical forces also has an outer dimension when flexed, and/or may even have a portion which flexes outwardly, such that the implantable medical device presents a cross-sectional dimension or area sufficient to retain the implantable medical device in place with respect to the deployment site.

In view of the above, an implantable medical device formed in accordance with various principles of the present disclosure is sized, shaped, configured, and/or dimensioned to withstand forces applied thereto, such as to flex, absorb, ride with, etc., such forces, without impacting the ability of the retention member to retain the implantable medical device in place with respect to the deployment site thereof. Such dimensions may be determined by one of ordinary skill in the art, generally depending on the nature and location of the deployment site and anatomical structures thereof, without undue experimentation.

In some embodiments, the retention member of an implantable medical device is configured to ride with anatomical forces applied thereto to be compressed by such forces, yet to return to an initial configuration upon such force subsiding. For instance, the retention member may be shiftable from an expanded configuration to a compressed configuration upon application of radially-inwardly directed forces thereto, yet may automatically return to the expanded configuration upon release or reduction of the force applied thereto. In some embodiments, the retention member is formed from a shape memory material returning the retention member to a neutral configuration, such as an expanded configuration (relative to the compressed configuration the retention member undergoes in response to anatomical forces applied thereto), resistant to migration.

In some embodiments, a retention member additionally or alternatively is movable with respect to the midsection of the implantable medical device. For instance, the retention member may be rotatable with respect to the midsection, and/or pivotable in and out of a plane transverse to the midsection. In some embodiments, the retention member is formed separately from the body section of the implantable medical device and coupled thereto, either directly or in directly, in a manner facilitating various degrees of freedom of movement of the retention member to ride with anatomical forces impacting the retention member. In some embodiments, the midsection is elastic to allow the retention member to be pulled generally longitudinally with respect to the midsection. The midsection may be formed to limit, occlude, exclude, etc., flow of materials therethrough, depending on the nature of the treatment for which the implantable medical device is deployed.

Various embodiments of implantable medical devices, systems, and methods will now be described with reference to examples illustrated in the accompanying drawings. Reference in this specification to "one embodiment," "an embodiment," "some embodiments," "other embodiments," etc. indicates that one or more particular features, structures, concepts, and/or characteristics in accordance with principles of the present disclosure may be included in connection with the embodiment. However, such references do not necessarily mean that all embodiments include the particular features, structures, concepts, and/or characteristics, or that an embodiment includes all features, structures, concepts, and/or characteristics. Some embodiments may include one or more such features, structures, concepts, and/or characteristics, in various combinations thereof. It should be understood that one or more of the features, structures, concepts, and/or characteristics described with reference to one embodiment can be combined with one or more of the features, structures, concepts, and/or characteristics of any of the other embodiments provided herein. That is, any of the features, structures, concepts, and/or characteristics described herein can be mixed and matched to create hybrid embodiments, and such hybrid embodiment are within the scope of the present disclosure. Moreover, references to "one embodiment," "an embodiment," "some embodiments," "other embodiments," etc. in various places in the specification are not necessarily all referring to the same embodiment, nor are separate or alternative embodiments necessarily mutually exclusive of other embodiments. It should further be understood that various features, structures, concepts, and/or characteristics of disclosed embodiments are independent of and separate from one another, and may be used or present individually or in various combinations with one another to create alternative embodiments which are considered part of the present disclosure. Therefore, the present disclosure is not limited to only the embodiments specifically described herein, as it would be too cumbersome to describe all of the numerous possible combinations and subcombinations of features, structures, concepts, and/or characteristics, and the examples of embodiments disclosed herein are not intended as limiting the broader aspects of the present disclosure. The following description is of illustrative examples of embodiments only, and is not intended as limiting the broader aspects of the present disclosure.

It will be appreciated that common features are identified by common reference elements and, for the sake of brevity and convenience, and without intent to limit, the descriptions of the common features are generally not repeated. For purposes of clarity, not all components having the same reference number are numbered. Moreover, a group of similar elements may be indicated by a number and letter, and reference may be made generally to one or such elements or such elements as a group by the number alone (without including the letters associated with each similar element). It will be appreciated that, in the following description, elements or components similar among the various illustrated embodiments with reference numbers greater than 100 are generally designated with the same reference numbers increased by a multiple of 100 and redundant description is generally omitted for the sake of brevity. Moreover, certain features in one embodiment may be used across different embodiments and are not necessarily individually labeled when appearing in different embodiments.

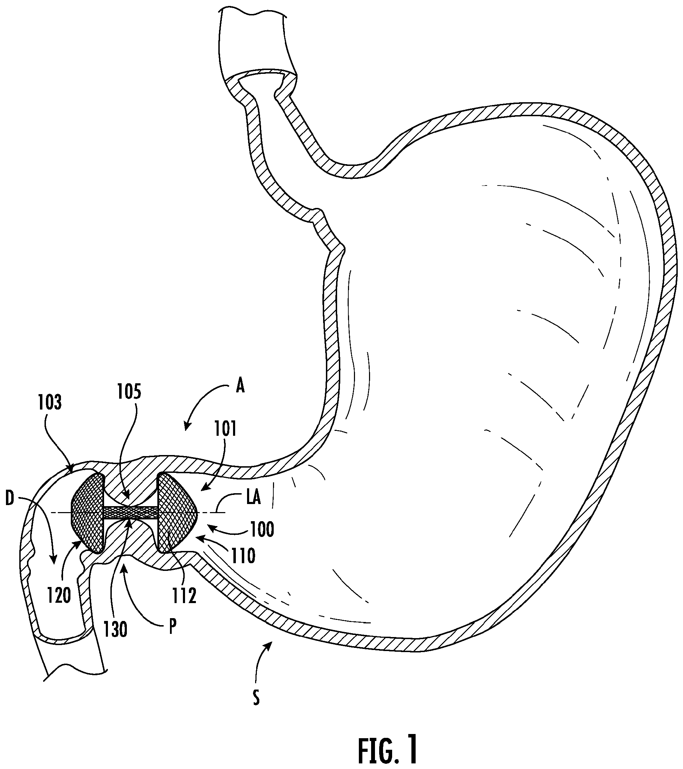

Turning now to the drawings, an example of an embodiment of an implantable medical device **100** is illustrated in **FIG. 1** in an example of an embodiment of a gastrointestinal system, though other deployment sites are within the scope and content of the present disclosure. More particularly, the illustrated example of an embodiment of an implantable medical device **100** includes a first end **101** positioned within a stomach **S,** a second end **103** positioned within a duodenum **D,** and a midsection **105** positioned within a pylorus **P.** A first retention member **110** extends from the first end **101** of the implantable medical device **100** radially outwardly with respect to the longitudinal axis **LA** of the implantable medical device **100** and/or midsection **105** (referenced herein simply as the longitudinal axis **LA** for the sake of convenience without intent to limit, unless explicitly stated). In the illustrated example of an embodiment, the first retention member **110** is configured to engage with a tissue wall of the stomach **S** (e.g., the antrum thereof) surrounding the entry into the pylorus **P.** A second retention member **120** extends from the second end **103** of the implantable medical device **100** radially outwardly with respect to the longitudinal axis **LA** and is configured to engage with tissue wall of the duodenum **D.** Although the second retention member **120** is illustrated as generally similar to the first retention member **110,** it will be appreciated that the second retention member **120** may have a different configuration than the first retention member **110,** as will be discussed in further detail below. The extent of the illustrated implantable medical device **100** along the midsection **105** and between the retention members **110, 120** is referenced herein as the body **130** of the implantable medical device **100** without intent to limit. It will be appreciated that a "body" of an implantable medical device **100** formed in accordance with various principles of the present disclosure may be considered to include or not include the retention members **110, 120,** the present disclosure not being restricted in this aspect.

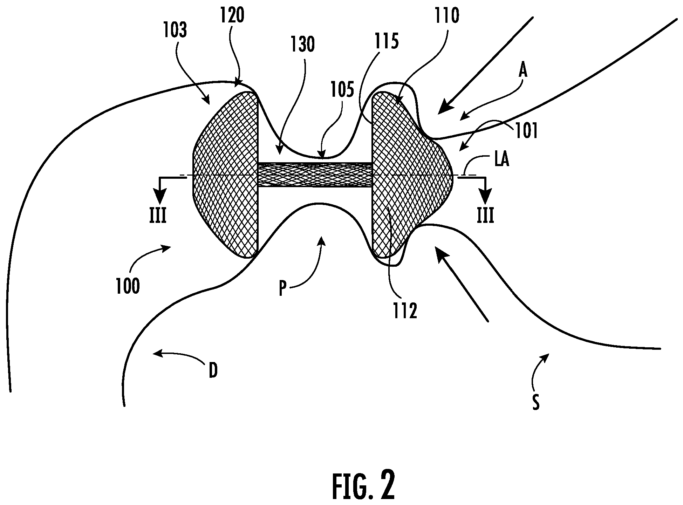

An implantable medical device **100** of the present disclosure, and particularly one or both of the retention members **110** thereof, may be subjected to regular forces, such as anatomical forces (internally and/or externally generated). For example, the illustrated implantable medical device **100** may be subjected to radially-inwardly-directed biological / anatomical forces. More particularly, when deployed in the environment illustrated in **FIG. 1****,** at least the first retention member **110** is regularly subjected to peristaltic waves from the stomach **S,** particularly, the antrum **A.** In accordance with various principles of the present disclosure, at least the first retention member **110** (within the anatomical site exhibiting regular and/or strong forces on the implantable medical device **100,** such as the antrum A) presents a convex tissue-contacting surface **112** for engaging and anchoring the implantable medical device **100** with respect to the deployment site, as illustrated in **FIG. 1** and **FIG. 2****.** More particularly, in contrast with prior implantable medical devices deployed in generally dynamic anatomical sites such as is the pylorus **P,** the tissue-contacting surface **112** does not present sharp or angular edges (e.g., generally clearly-defined edges, such as formed by surfaces joined at less than about 145° with respect to each other) for contact with tissue which may exert localized forces on such tissue-contacting surface **112.** Instead, the tissue-contacting surface **112** presents a generally convex surface extending radially-outwardly from the longitudinal axis **LA.** Moreover, the first retention member **110** is sufficiently compliant and the tissue-contacting surface **112** thereof extends a sufficient distance radially-outwardly from the longitudinal axis **LA** and along the longitudinal axis **LA** and towards the midsection **105** to present a convex surface area with a sufficiently large radius of curvature to flex and dissipate forces applied thereto. As may be appreciated, the size, shape, configuration, and/or dimensions of the retention member **110** and the tissue-contacting surface **112** thereof, and/or stiffness and/or flexibility of the retention member **110,** may be selected such that the tissue-contacting surface **112** is contacted over a sufficient area to dissipate forces impacting the retention member **110.** Such size, shape, configuration, and/or dimensions, and/or stiffness / flexibility may be determined by one of ordinary skill in the art such as with reference to the nature of the deployment site without undue experimentation. It will be appreciated that references herein to various features and characteristics of the first retention member of an implantable medical device formed in accordance with various principles of the present disclosure may be applied to the second retention member as well.

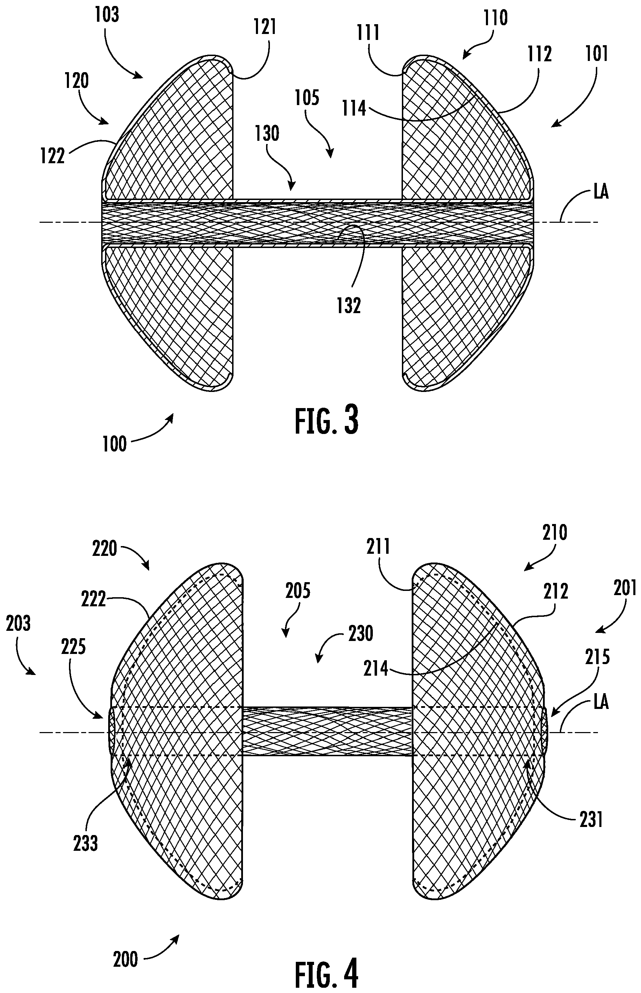

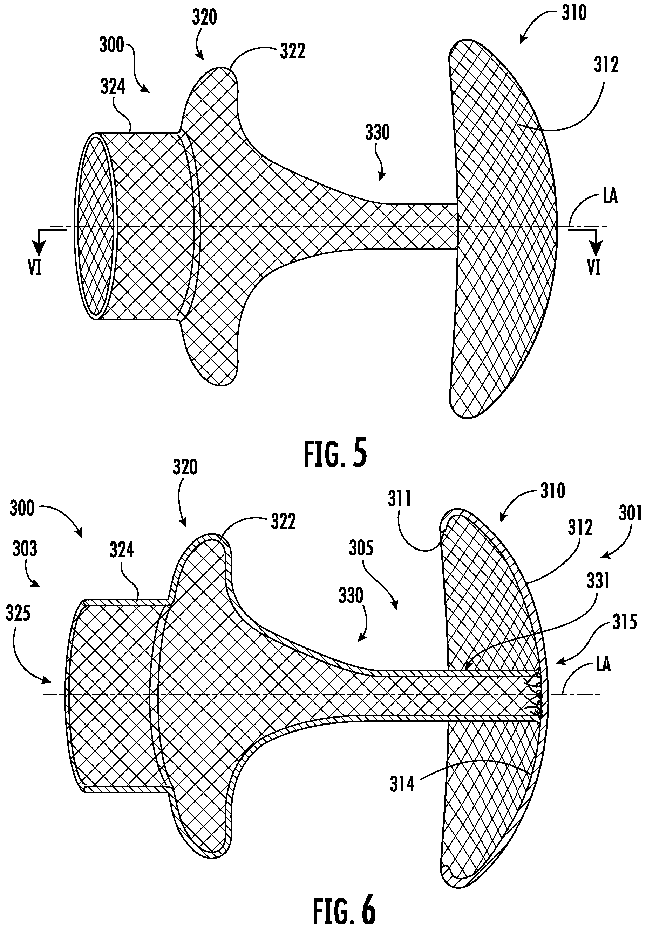

In accordance with further aspects of the present disclosure, the generally convex tissue-contacting surface **112** of a retention member **110** formed in accordance with various principles of the present disclosure is configured to resist inversion of the retention member **110** and thereby to resist migration which may occur if the retention member **110** is inverted with a typically thereby narrower dimension in a direction transverse to the longitudinal axis **LA.** For instance, the retention member **110** may be configured such as to form a generally umbrella-type configuration. For instance, the outer tissue-contacting surface **112** of the retention member **100** is convex, whereas the interior surface may be generally concave, such as illustrated in **FIG. 3** and **FIG. 6****,** and described in further detail below. As with umbrellas, such shape generally resists inversion. As may be appreciated by those of ordinary skill in the art, dimensions, proportions, etc., tensile strength, material composition, cross sectional shape as well as bending stiffness and/or flexibility of the retention member 110 may be selected, such as with reference to the ultimate deployment site thereof, to assure the desired resistance to inversion.

An example of an embodiment of a first retention member **110** formed in accordance with various principles of the present disclosure is illustrated in **FIG. 2****.** As may be appreciated, the generally convex tissue-contacting surface **112** has sufficient area to withstand forces (indicated by the generally inwardly directed arrows) applied thereto from the deployment site (in this case the antrum **A** of the stomach **S).** Moreover, the first retention member **110** and tissue-contacting surface **112** are sufficiently flexible / compliant to generally conform with the shape of the tissue wall applying the force and/or to otherwise dissipate ("ride with") the forces applied thereto rather than to resist such forces. As such, forces applied to the implantable medical device **100** are dissipated, and forces and stresses do not concentrate at particular regions of the first retention member **110** as may occur with prior retention members of similar implantable medical devices. Moreover, if the first retention member **110** is sufficiently compliant, then the tissue-contacting surface **112** may withstand forces applied thereto without propagating such forces to the free end **115** of the first retention member **110.** It will be appreciated that reference to the free end **115** is intended to encompass reference to the peripheral free edge of the first retention member **110.**

In some embodiments, an implantable medical device **100** is formed from a plurality of strands or wires or filaments or ribbons (such terms being used interchangeably herein without intent to limit) which are braided or woven or twisted or wrapped or intertwined or knitted or looped (e.g., bobbinet-style) or knotted or otherwise to form the wall of the implantable medical device **100.** The free ends of the filaments forming the implantable medical device **100** may be welded to retain the filaments in place with respect to one another. For the sake of convenience, and without intent to limit, reference is made generally to formation of an implantable medical device with interwoven filaments to refer to the general manner of forming an implantable medical device from multiple interconnected elements. In contrast, an implantable medical device **100** may alternatively be formed from a single, monolithic element such as a laser cut tube, cut or otherwise shaped into the desired configuration. The filaments may be formed of generally biocompatible materials such as a variety of appropriate metals (e.g., nickel-titanium alloy, such as Nitinol, or another generally shapable metal, such as stainless steel) or polymers or combinations thereof known to those of ordinary skill in the art. In some embodiments, the material of the implantable medical device is a shape memory and/or heat formable material allowing the retention member a degree of resiliency so that the retention member does not plastically deform and returns to an expanded configuration to resist migration from the deployment site and thereby to retain the implantable medical device in place.

A reduction of forces propagated to the free end **115** of the first retention member **110** generally reduces forces which may affect the integrity of the welds of filaments forming a woven implantable medical device **100** and thereby affecting the integrity of the implantable medical device **100.** As such, a configuration of a compliant first retention member **110** and/or a first retention member **110** with a convex tissue-contacting surface **112** in accordance with various principles of the present disclosure reduce stresses which may otherwise affect the structural integrity of the wall thereof.

As may be appreciated with reference to the cross-sectional view in **FIG. 3****,** along line **III-III** of the example of an embodiment of an implantable medical device **100** illustrated in **FIG. 2****,** at least the first retention member **110** may also have a generally concave inner surface **114** generally facing in a direction opposite the direction in which the tissue-contacting surface **112** faces. The combination of a generally convex tissue-contacting surface **112** and a generally concave inner surface **114** permits the first retention member **110** to resist inversion, similar to a manner in which an umbrella may resist inversion. Such configuration may counterbalance / offset various more compliant features of the first retention member **110.**

An implantable medical device **100** formed in accordance with various principles of the present disclosure may be formed from a generally tubular member defining the tubular body **130.** To form one or both of the retention members **110, 120,** an end region of the tubular member forming the tubular body **130** may be inverted and pulled in a direction towards the other end of the tubular member, into a first retention member **110** such as illustrated in **FIG. 3****.** More particularly, the free end or circumferential edge of the tubular member may be inverted and pulled a sufficient distance toward a midsection thereof (between the ends of the tubular member) to form a retention member **110, 120** on either end of a body **130** which resists returning to the initial tubular configuration. It will be appreciated that the ends of the body **130** formed from the tubular member are not necessarily the ends of the tubular member from which the body **130** is formed, as the ends of the tubular member may be free ends and thus may be define free ends of the retention members **110, 120** formed by inverting the free ends of the tubular member. In some embodiments, the inverted ends of the tubular member are also shaped and formed to define a generally convex tissue-contacting surface **112, 212** such as illustrated in **FIG. 3****.** The free end **111, 121** of the respective retention members **110, 120** is extended a sufficient distance towards the opposite retention member **120, 110** to provide sufficient area for the respective tissue-contacting surfaces **112, 122** to anchor the implantable medical device **100** as well as to provide sufficient structure (length, bending stiffness, etc.) to resist inversion or "unrolling" of the first retention member **110** to its starting position (which may be less resistant to migration of the implantable medical device **100).** In some embodiments, the free ends **111, 121** may be directed inwardly towards the longitudinal axis **LA.** In embodiments in which the implantable medical device **100** is formed from weaving or otherwise interengaging filaments, the filaments may be woven to form a generally tubular member from which the implantable medical device **100** may be formed. The end of the tubular member may then be inverted, and optionally also stretched, into the inverted umbrella-like configuration illustrated in **FIG. 3****.**

It will be appreciated that even if the body **130** of an implantable medical device **100** formed in accordance with various principles of the present disclosure is tubular and defines a lumen therethrough, such as illustrated in **FIG. 2** and **FIG. 3****,** the lumen need not be configured to allow passage of material therethrough readily if at all. In some embodiments the lumen may be occluded such as to inhibit or to prevent flow of materials therethrough. For instance, in the example of an embodiment illustrated in **FIG. 2** and **FIG. 3****,** the inner diameter of the lumen **132** through the body **130** of the implantable medical device **100** may be narrowed, constricted, or occluded to reduce or to prevent flow of materials therethrough. In the example of an embodiment illustrated in **FIG. 1****,** occlusion of the implantable medical device **100** may be desirable to inhibit or to prevent flow of gastric materials from the stomach **S** into the duodenum **D** (such as in conjunction with bariatric treatments, including formation of a gastric bypass formed between the stomach **S** and the jejunum).

The retention members which anchor the implantable medical device in place with respect to the deployment site may be formed separately from the body portion which extends across a deployment site such as an anatomical passage. Additionally or alternatively, the retention members and the body may be formed in a different manner, such as from different materials with optionally different properties.

An example of another embodiment of an implantable medical device **200** formed in accordance with various principles of the present disclosure with at least one generally compliant retention member **210, 220** is illustrated in **FIG. 4****.** The compliant retention members **210, 220** may have any of the properties of the retention members **110, 120** described above, in any combination thereof, which allow compliant riding with forces applied thereto while resisting migration and retaining the implantable medical device in place at its deployment site. Accordingly, for the sake of brevity, reference is made to the above descriptions of properties of a compliant retention member formed in accordance with various principles of the present disclosure as applicable to retention members **210, 220** of **FIG. 4****.** In the illustrated example of an embodiment, the tissue-contacting surfaces **212, 222** of the retention members **210, 220** extend from a midregion **215, 225** positioned generally along the longitudinal axis **LA,** radially outwardly from the longitudinal axis **LA,** and generally longitudinally toward the midsection **235** of the body **230** and the opposite retention member **220, 210.** The retention members **210, 220** may be generally bowl-shaped to present a generally convex tissue-contacting surfaces **212, 222** to tissue at the deployment site to absorb forces applied thereto.

In the example of an embodiment of an implantable medical device **200** illustrated in **FIG. 4****,** one or both of the retention members **210, 220** are formed separately from the body **230** of the implantable medical device **200** extending therebetween. As such, at least one of the retention members **210, 220** is more readily movable with respect to the body **230** than in prior implantable medical devices formed for similar purposes. For instance, at least one of the retention members **210, 220** may be rotatable and/or pivotable (e.g., in and out of a plane transverse to the longitudinal axis **LA)** with respect to the body **230.** The ends **231, 233** of the body **230** may be coupled to the retention members **210, 220** in any of a variety of manners allowing relative movement between the body **230** and the retention members **210, 220.** The ends of the body **230** may be bonded, looped, interwoven, hooked, or otherwise engaged with the retention members **210, 220** ¸either directly or with another element (e.g., a suture), to hold the components of the implantable medical device **200** together, such as depending on the manner in which the retention members **210, 220** and body **230** are formed (as described in further detail below). In some embodiments, the ends **231, 233** of the body **230** are coupled with generally central midregions **215, 225** of the retention members **210, 220.** In some embodiments, the body **230** is insertable into a generally central region of the retention members **210, 220** to allow rotation of the retention members **210, 220** with respect to the body **230,** yet to prevent separation of the retention members **210, 220** from the body **230.** For instance, the free ends of the body **230** may be enlarged (e.g., after being inserted through the retention members **210, 220)** to maintain a connection with the retention members **210, 220.** It will be appreciated that the manner of connecting the body **230** with the retention members **210, 220** may be a generally flexible, generally not rigid, connection, thereby generally avoiding stress concentrations and allowing a degree of movement and "riding" with anatomical forces applied thereto.

Additionally or alternatively, different materials may be used to form the retention members **210, 220** and the body **230.** In some embodiments, the body **230** of the implantable medical device **200** of **FIG. 4** may be formed of an elastic material allowing stretching to accommodate anatomical forces impacting the implantable medical device **200** rather than resisting such forces. As such, not only are the retention members **210, 220** able to "ride" with anatomical forces rather than resist such forces, but the body **230** may also "ride" with anatomical forces impacting the implantable medical device **200.**

Additionally or alternatively, the retention members **210, 220** and the body **230** of the example of an embodiment of an implantable medical device **200** illustrated in **FIG. 4** may be formed in different manners. For instance, the retention members **210, 220** may be formed from woven filaments, similar to the retention members **110, 120** of the implantable medical device **100** illustrated in **FIG. 2** and **FIG. 3****.** In contrast, instead of being formed from woven filaments, the body **230** of the implantable medical device **200** illustrated in **FIG. 4** may be formed from an elastic rod or even an elastic band (e.g., similar to a rubber band) or tether element or the like. The ends **231, 233** of the body **230** (e.g., ends of filaments forming the body **230** and/or ends of a band forming the body **230)** may be looped about elements, such as filaments, forming the retention members **210, 220** to hold the body **230** and retention members **210, 220** together in a manner allowing relative movement therebetween.

As noted above, the retention members of an implantable medical device formed in accordance with various principles of the present disclosure need not have the same configurations. For instance, the example of an embodiment of an implantable medical device **300** illustrated in **FIG. 5** and **FIG. 6** may have differently shaped retention members **310, 320.** The first retention member **310** may be bowl-shaped and formed similar to the bowl-shaped retention members **110, 210** described above and illustrated in **FIG. 2****,** **FIG. 3,** and **FIG. 4****.** For instance, the first retention member **310** may extend from a generally central region **315** at the first end **301** of the body **330,** radially outwardly from the longitudinal axis **LA,** and towards the midsection **305** of the body **330.** Moreover, the first retention member **310** may be compliant and flexible to flex inwardly in response to radially inwardly forces exerted thereon and thereby to conform to the anatomical structure at which the implantable medical device **300** is deployed. The first retention member **310** preferably does not plastically deform, and is able to return to an expanded configuration upon reduction or withdrawal of the anatomical forces applied thereto. As such, the first retention member **310** is configured to "ride" radially-inwardly directed forces, such as peristaltic waves, impacting the implantable medical device **300.** Like the retention members **110, 210** described above, the first retention member **310** is sized, shaped, configured, and/or dimensioned to resist migration of the implantable medical device **300** even when being compressed or otherwise deformed in response to forces applied thereto. It will be appreciated that such properties of a retention member are applicable to the above-described retention members, and the above-described properties of previously described examples of embodiments of retention members are applicable to the retention member **310** illustrated in **FIG. 5** and **FIG. 6****.**

In deployment sites in which the second retention member **320** is subjected to anatomical forces of a different nature than those impacting the first retention member **310,** the second retention member **320** may have a different shape and configuration than that of the first retention member **310.** For instance, the example of an embodiment of an implantable medical device **300** illustrated in **FIG. 5** and **FIG. 6** is configured to be implanted across a pylorus **P** with the second retention member **320** extending into a duodenum **D (****FIG.1****).** The second retention member **320** includes a tissue-contacting surface **322** extending radially-outwardly from the longitudinal axis **LA** and configured to engage the duodenum **D** to hold the implantable medical device **300** in place with respect to the duodenum **D** and against migration. However, because the deployment site of the second retention member **320** is the duodenum **D** rather than the stomach **S,** the second retention member **320** may be more elongated (for example, along the longitudinal axis **LA)** than the first retention member **310,** e.g., with an extended generally cylindrical tissue-contacting surface **324.** Such elongated configuration of the second retention member **320** may distribute and dissipate peristaltic forces from the duodenum **D** along the length thereof better than the generally shorter retention member **310** configured for placement on the gastric side of the pylorus **P.** In some embodiments, the diameter of the tissue-contacting surface **322** of the second retention member **320** is closer to the diameter of the generally cylindrical tissue-contacting surface **324** than as illustrated in **FIG. 5** and **FIG. 6****,** and, in some embodiments, these diameters may even be substantially the same. Additionally or alternatively, the transition from the body **330** to the second retention member **320** may be gradual, as illustrated in **FIG. 5** and **FIG. 6****,** or more angular (e.g., similar to the transition to the first retention member **310),** depending on the anatomical structure in which the second retention member **320** is to be deployed. Moreover, the antrum **A** typically exerts stronger peristaltic forces than exerted by the duodenum **D,** thereby requiring modifications to the gastric retention member **310** (such as the features described herein) not necessarily required in the duodenal retention member **320.**

The body **330** and retention members **310, 320** of the implantable medical device **300** illustrated in **FIG. 5** and **FIG. 6** may be formed as a single component or from separate components coupled together. For instance, the body **330** of the implantable medical device **300** may be formed from a tubular member, with the ends thereof expanded to form the retention members **310, 320** (such as described with respect to the implantable medical device **100** illustrated in **FIG. 2** and **FIG. 3****).** More particularly, the body **330** may be formed from a generally tubular member (e.g., from interwoven filaments) with an end along the first end **301** of the implantable medical device **300** inverted to form the first retention member **310** with the free end **315** thereof spaced inwardly from the first end **301** of the implantable medical device **300** and towards the midsection **305** of the implantable medical device **300.** The body **330** may be expanded along the second end **303** of the implantable medical device **300** to form the second retention member **320.** Alternatively, the body **330** and the second retention member **310** of the implantable medical device **300** illustrated in **FIG. 5** may be formed together, but separately from the first retention member **310.** For instance, the body **330** and the second retention member **310** may be formed from a tubular member expanded at one end to form the second retention member **320.** The first retention member **310** may be separately formed in a bowl-shaped configuration with a convex tissue-contacting surface **312,** and then coupled with the body **330** and the second retention member **310.** The first retention member **310** may be formed from interwoven filaments or in any other manner allowing compliant and resilient compression in response to radially-inwardly directed forces, and the ability to return to an expanded configuration upon forces applied thereto subsiding, and to resist migration of the implantable medical device **300** from its deployment site, such as in manners described above.

The formation of the first retention member **310** of the implantable medical device **300** illustrated in **FIG. 5** and **FIG. 6** separate from the body **330** may afford greater degrees of freedom of movement to the first retention member **310** than may be afforded if formed integrally with and as part of the body **330.** Such freedom of movement may allow the first retention member **310** to ride with anatomical forces impacting the implantable medical device **300** with less stress on the first retention member **310** than withstood by prior art devices used for similar purposes. The body **330** and the first retention member **310** may be connected in a variety of manners affording multiple degrees of freedom of movement. For instance, if the body **330** and the first retention member **310** are formed from interwoven filaments, the free ends of filaments forming the body **330** may be looped around or otherwise interwoven or interegaged with the first retention member **310** (e.g., with filaments forming the first retention member **310)** to couple the body **330** and the first retention member **310** together. For instance, as illustrated in further detail in **FIG. 6****,** ends of filaments at the first end **311** of the body **330** may be looped around filaments forming the first retention member **310** to couple the body **330** to the first retention member **310.** Such connection allows rotation and/or pivoting of the first retention member **310** with respect to the body **330,** such as in manners described herein with respect to various retention members formed in accordance with various principles of the present disclosure (such as the implantable medical device **200** illustrated in **FIG. 4****).** In some embodiments, the first retention member **310** may be coated with a biocompatible material, such as silicone, which may bond the free ends of the filaments of the body **330** with respect to the first retention member **310** and/or inhibit tissue ingrowth with respect to the first retention member **310** (thereby facilitating movement with respect to tissue at the deployment site), and/or inhibit or prevent flow of materials therethrough. Alternatively, if the first retention member **310** is not formed with interwoven filaments, then the body **330** may be coupled thereto in other manners known to those of ordinary skill in the art, such as described above with reference to the example of an embodiment illustrated in **FIG. 4****.**

As noted above, it will be appreciated that various features of the above-described examples of embodiments can be interchanged with those of others of the above-described examples of embodiments, such features being arranged and operating in substantially the same or similar manners. Accordingly, common elements with common functions have been indicated with the same reference characters differing in value by 100, with descriptions of features of one embodiment being generally applicable to similar features of other embodiments, such descriptions not being repeated for the sake of brevity and convenience, and without intent to limit.

Although embodiments of the present disclosure may be described with specific reference to medical devices and systems and procedures for treating the gastrointestinal system, it should be appreciated that such medical devices and methods may be used to treat tissues of the abdominal cavity, digestive system, urinary tract, reproductive tract, respiratory system, cardiovascular system, circulatory system, and the like. It will be appreciated that reference to a body passage includes naturally-existing passages (e.g., the colon) as well as medically-created passages (e.g., a passage created with the use of a medical instrument, and not existing without medical intervention) or otherwise.

Various further benefits of the various aspects, features, components, and structures of an implantable medical device such as described above, in addition to those discussed above, may be appreciated by those of ordinary skill in the art.

In the foregoing description and the following claims, the following will be appreciated. The phrases "at least one", "one or more", and "and/or", as used herein, are open-ended expressions that are both conjunctive and disjunctive in operation. The terms "a", "an", "the", "first", "second", etc., do not preclude a plurality. For example, the term "a" or "an" entity, as used herein, refers to one or more of that entity. As such, the terms "a" (or "an"), "one or more", and "at least one" can be used interchangeably herein. As used in this specification and the appended claims, the term "or" is generally employed in its sense including "and/or" unless the content clearly dictates otherwise. As used herein, the conjunction "and" includes each of the structures, components, features, or the like, which are so conjoined, unless the context clearly indicates otherwise, and the conjunction "or" includes one or the others of the structures, components, features, or the like, which are so conjoined, singly and in any combination and number, unless the context clearly indicates otherwise. All directional references (e.g., proximal, distal, upper, lower, upward, downward, left, right, lateral, longitudinal, front, back, top, bottom, above, below, vertical, horizontal, radial, axial, clockwise, counterclockwise, and/or the like) are only used for identification purposes to aid the reader's understanding of the present disclosure, and/or serve to distinguish regions of the associated elements from one another, and do not limit the associated element, particularly as to the position, orientation, or use of this disclosure. Connection references (e.g., attached, coupled, connected, engaged, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another.

The following claims are hereby incorporated into this Detailed Description by this reference, with each claim standing on its own as a separate embodiment of the present disclosure. In the claims, the terms "comprises", "comprising", "includes", and "including" do not exclude the presence of other elements, components, features, groups, regions, integers, steps, operations, etc. Additionally, although individual features may be included in different claims, these may possibly advantageously be combined, and the inclusion in different claims does not imply that a combination of features is not feasible and/or advantageous. In addition, singular references do not exclude a plurality. Reference signs in the claims are provided merely as a clarifying example and shall not be construed as limiting the scope of the claims in any way.

## Claims

1. An implantable medical device (100, 200, 300) comprising:
a body (130, 230, 330) having a first end (101, 201, 301), a second end (103, 203, 303), and a midsection (105, 235, 305) therebetween;
a first retention member (110, 210, 310) extending from the first end (101, 201, 301) of said body (130, 230, 330); and
a second retention member (120, 220, 320) extending from the second end (103, 203, 303) of said body (130, 230, 330);
wherein:
said first retention member (110, 210, 310) extends radially outwardly from the first end (101, 201, 301) of said body (130, 230, 330) and toward the midsection (105, 235, 305) of said body (130, 230, 330) to define a tissue-contacting surface (112, 212, 312) with a surface area and flexibility sufficient to absorb radially-inwardly directed anatomical forces applied thereto;
said first retention member (110, 210, 310) is configured to resist being inverted into a configuration with a free end (111, 211, 311) thereof extending away from the first end (101, 201, 301) and the midsection (105, 235, 305) of said body (130, 230, 330); and
said body (130, 230, 330) is tubular and defines a lumen (132) therethrough, **characterised in that** the lumen (132) is configured to reduce or to prevent flow of materials therethrough.

2. The implantable medical device (100, 200, 300) of claim 1, wherein said first retention member (110, 210, 310) is formed of a compliant material capable of flexing with radially-inwardly directed anatomical forces applied thereto.

3. The implantable medical device (100, 200, 300) of claim 2, wherein said first retention member (110, 210, 310) is compressible from an initial expanded configuration in response to application of radially-inwardly forces applied thereto, and is formed of a resilient material returning said first retention member (110, 210, 310) to the expanded configuration upon the force subsiding.

4. The implantable medical device (100, 200, 300) of claim 3, wherein said first retention member (110, 210, 310) presents a convex tissue-contacting surface (112, 212, 222, 312) to tissue at the deployment site, and is configured and dimensioned to resist migration from the deployment site.

5. The implantable medical device (100, 200, 300) of claim 1, wherein said tubular body (130, 230, 330) is formed of a tubular member, and at least said first retention member (110, 210, 310) is formed by inverting an end of the tubular member and extending the free end (111, 211, 311) of the inverted end toward the midsection (105, 235, 305) of the tubular member a sufficient distance to cause the inverted end of the tubular member to resist returning to its initial uninverted tubular configuration.

6. The implantable medical device (100, 200, 300) of claim 5, wherein said tubular member is formed from a plurality of interwoven filaments.

7. The implantable medical device (100, 200, 300) of claim 1, wherein said first retention member (110, 210, 310) is formed from a bowl-shaped element separate from said body (130, 230, 330).

8. The implantable medical device (100, 200, 300) of claim 7, wherein said first retention member (110, 210, 310) is movable with respect to said body (130, 230, 330).

9. The implantable medical device (100, 200, 300) of claim 7, wherein said body (130, 230, 330) is formed from an elastic material allowing said first retention member (110, 210, 310) to be moved away from said second retention member (120, 220, 320).

10. The implantable medical device (100, 200, 300) of any one of claims 1-9, wherein the tissue-contacting surface (112, 212, 312) of said first retention member (110, 210, 310) is convex.

11. The implantable medical device (100, 200, 300) of any one of claims 1-10, wherein the
device is configured to be implanted with respect to a pylorus of a patient, wherein:
the first retention member (110, 210, 310) is a gastric retention member; and
the second retention member (120, 220, 320) is a duodenal retention member;
said gastric retention member has a free edge of said gastric retention member spaced from the first end (101, 201, 301) of said body (130, 230, 330) in a direction toward the midsection (105, 235, 305) of said body (130, 230, 330) a sufficient extent to remain in such configuration when said implantable medical device (100, 200, 300) is implanted with respect to the patient's pylorus with said gastric retention member positioned within the patient's stomach; and
said gastric retention member is resiliently compliant to contract from an expanded configuration to a compressed configuration upon application of peristaltic forces thereto from the antrum of the patient's stomach, and to return to the expanded configuration upon passing of a peristaltic wave from said gastric retention member.

12. The implantable medical device (100, 200, 300) of claim 11, wherein said gastric retention member presents a convex tissue-contacting surface (112, 212, 222, 312) to the antrum of the patient's stomach.

13. The implantable medical device (100, 200, 300) of claim 11, wherein:
said implantable medical device (100, 200, 300) is formed from a tubular member having a first end (101, 201, 301), a second end (103, 203, 303), and a midsection (105, 235, 305) therebetween; and
said gastric retention member is formed by inverting the first end (101, 201, 301) of the tubular member and pulling the free end (111, 211, 311) thereof towards the second end (103, 203, 303) of the tubular member a sufficient distance to remain in the inverted configuration when implanted with respect to the antrum of the patient's stomach and when compressed by a peristaltic wave of the antrum.

14. The implantable medical device (100, 200, 300) of claim 11, wherein said body (130, 230, 330) and at least said gastric retention member are formed separately and coupled together to allow relative movement therebetween.

## Patentansprüche

1. Implantierbare medizinische Vorrichtung (100, 200, 300), aufweisend:
einen Körper (130, 230, 330), der ein erstes Ende (101, 201, 301), ein zweites Ende (103, 203, 303) und einen dazwischen angeordneten Mittenabschnitt (105, 235, 305) hat;
ein sich vom ersten Ende (101, 201, 301) des Körpers (130, 230, 330) erstreckendes erstes Halteelement (110, 210, 310); und
ein sich vom zweiten Ende (103, 203, 303) des Körpers (130, 230, 330) erstreckendes zweites Halteelement (120, 220, 320), wobei:
sich das erste Halteelement (110, 210, 310) vom ersten Ende (101, 201, 301) des Körpers (130, 230, 330) radial nach außen und in Richtung zum Mittenabschnitt (105, 235, 305) des Körpers (130, 230, 330) erstreckt, um eine Gewebekontaktfläche (112, 212, 312) mit einem Oberflächenmaß und einer Flexibilität zu definieren, die ausreichen, um darauf ausgeübte, radial nach innen gerichtete anatomische Kräfte zu absorbieren,
wobei das erste Halteelement (110, 210, 310) dafür konfiguriert ist, einem Umwenden in eine Konfiguration zu widerstehen, bei der sich ein freies Ende (111, 211, 311) davon vom ersten Ende (101, 201, 301) und vom Mittenabschnitt (105, 235, 305) des Körpers (130, 230, 330) weg erstreckt, und
wobei der Körper (130, 230, 330) rohrförmig ist und ein sich durch ihn erstreckendes Lumen (132) definiert,
**dadurch gekennzeichnet, dass**
das Lumen (132) dafür konfiguriert ist, Material-Durchfluss dort hindurch zu reduzieren oder zu verhindern.

2. Implantierbare medizinische Vorrichtung (100, 200, 300) nach Anspruch 1, wobei das erste Halteelement (110, 210, 310) aus einem nachgiebigen Material ausgebildet ist, das in der Lage ist, sich zu biegen, wenn radial nach innen gerichtete anatomische Kräfte darauf ausgeübt werden.

3. Implantierbare medizinische Vorrichtung (100, 200, 300) nach Anspruch 2, wobei das erste Halteelement (110, 210, 310) in Antwort auf das Ausüben von darauf ausgeübten, radial nach innen gerichteten Kräften von einer anfänglichen expandierten Konfiguration komprimierbar ist und aus einem elastischen Material ausgebildet ist, das das erste Halteelement (110, 210, 310) in die expandierte Konfiguration zurückkehren lässt, sobald die Kraft nachlässt.

4. Implantierbare medizinische Vorrichtung (100, 200, 300) nach Anspruch 3, wobei das erste Halteelement (110, 210, 310) einem Gewebe an der Entfaltungsstelle eine konvexe Gewebekontaktfläche (112, 212, 222, 312) präsentiert und dafür konfiguriert und dimensioniert ist, einer Migration von der Entfaltungsstelle zu widerstehen.

5. Implantierbare medizinische Vorrichtung (100, 200, 300) nach Anspruch 1, wobei der rohrförmige Körper (130, 230, 330) aus einem rohrförmigen Element ausgebildet ist und zumindest das erste Halteelement (110, 210, 310) dadurch gebildet wird, dass ein Ende des rohrförmigen Elements umgewendet und das freie Ende (111, 211, 311) des umgewendeten Endes in Richtung zum Mittenabschnitt (105, 235, 305) des rohrförmigen Elements hin um eine ausreichende Strecke verlängert wird, um zu veranlassen, dass das umgewendete Ende des rohrförmigen Elements einem Zurückkehren in seine ursprüngliche, nicht umgewendete rohrförmige Konfiguration widersteht.

6. Implantierbare medizinische Vorrichtung (100, 200, 300) nach Anspruch 5, wobei das rohrförmige Element aus mehreren miteinander verwobenen Filamenten gebildet ist.

7. Implantierbare medizinische Vorrichtung (100, 200, 300) nach Anspruch 1, wobei das erste Halteelement (110, 210, 310) aus einem schalenförmigen Element ausgebildet ist, das vom Körper (130, 230, 330) getrennt ist.

8. Implantierbare medizinische Vorrichtung (100, 200, 300) nach Anspruch 7, wobei das erste Halteelement (110, 210, 310) bezüglich des Körpers (130, 230, 330) bewegbar ist.

9. Implantierbare medizinische Vorrichtung (100, 200, 300) nach Anspruch 7, wobei der Körper (130, 230, 330) aus einem elastischen Material ausgebildet ist, das es ermöglicht, dass das erste Halteelement (110, 210, 310) vom zweiten Halteelement (120, 220, 320) wegbewegt werden kann.

10. Implantierbare medizinische Vorrichtung (100, 200, 300) nach einem der Ansprüche 1 bis 9, wobei die Gewebekontaktfläche (112, 212, 312) des ersten Halteelements (110, 210, 310) konvex ist.

11. Implantierbare medizinische Vorrichtung (100, 200, 300) nach einem der Ansprüche 1 bis 10, wobei die Vorrichtung dafür konfiguriert ist, in Bezug auf einen Pylorus eines Patienten implantiert zu werden, wobei:
das erste Halteelement (110, 210, 310) ein gastrales Halteelement ist, und
das zweite Halteelement (120, 220, 320) ein duodenales Halteelement ist,
wobei das gastrale Halteelement einen freien Rand aufweist, der in Richtung zum Mittenabschnitt (105, 235, 305) des Körpers (130, 230, 330) in einem ausreichenden Maß vom ersten Ende (101, 201, 301) des Körpers (130, 230, 330) beabstandet ist, um in dieser Konfiguration zu verbleiben, wenn die implantierbare medizinische Vorrichtung (100, 200, 300) in Bezug auf den Pylorus des Patienten implantiert ist, wobei das gastrale Halteelement im Magen des Patienten positioniert ist; und
das gastrale Halteelement elastisch nachgiebig ist, so dass es sich, wenn peristaltische Kräfte vom Antrum des Magens des Patienten ausgeübt werden, von einer expandierten Konfiguration in eine komprimierte Konfiguration zusammenzieht und nach dem Passieren einer peristaltischen Welle vom gastralen Halteelement in die expandierte Konfiguration zurückkehrt.

12. Implantierbare medizinische Vorrichtung (100, 200, 300) nach Anspruch 11, wobei das gastrale Halteelement dem Antrum des Magens des Patienten eine konvexe Gewebekontaktfläche (112, 212, 222, 312) präsentiert.

13. Implantierbare medizinische Vorrichtung (100, 200, 300) nach Anspruch 11, wobei:
die implantierbare medizinische Vorrichtung (100, 200, 300) aus einem rohrförmigen Element ausgebildet ist, das ein erstes Ende (101, 201, 301), ein zweites Ende (103, 203, 303) und einen dazwischen angeordneten Mittenabschnitt (105, 235, 305) aufweist; und
das gastrale Halteelement gebildet wird durch Umwenden des ersten Endes (101, 201, 301) des rohrförmigen Körpers und Ziehen von dessen freiem Ende (111, 211, 311) in Richtung zum zweiten Ende (103, 203, 303) des rohrförmigen Körpers um eine ausreichende Strecke, so dass es in der umgewendeten Konfiguration verbleibt, wenn es in Bezug auf das Antrum des Magens des Patienten implantiert ist und wenn es durch eine peristaltische Welle des Antrums komprimiert wird.

14. Implantierbare medizinische Vorrichtung (100, 200, 300) nach Anspruch 11, wobei der Körper (130, 230, 330) und zumindest das gastrale Halteelement separat ausgebildet und miteinander verbunden sind, um eine Relativbewegung dazwischen zu ermöglichen.

## Revendications

1. Dispositif médical implantable (100, 200, 300) comprenant :
un corps (130, 230, 330) ayant une première extrémité (101, 201, 301), une deuxième extrémité (103, 203, 303) et une section médiane (105, 235, 305) entre elles ;
un premier élément de retenue (110, 210, 310) s'étendant à partir de la première extrémité (101, 201, 301) dudit corps (130, 230, 330) ; et
un deuxième élément de retenue (120, 220, 320) s'étendant à partir de la deuxième extrémité (103, 203, 303) dudit corps (130, 230, 330) ;
dans lequel :
ledit premier élément de retenue (110, 210, 310) s'étend radialement vers l'extérieur à partir de la première extrémité (101, 201, 301) dudit corps (130, 230, 330) et vers la section médiane (105, 235, 305) dudit corps (130, 230, 330) afin de définir une surface de contact avec le tissu (112, 212, 312) avec une surface et la flexibilité suffisante pour absorber les forces anatomiques dirigées radialement vers l'intérieur appliquées sur cette dernière ;
ledit premier élément de retenue (110, 210, 310) est configuré pour résister à l'inversion dans une configuration avec son extrémité libre (111, 211, 311) qui s'étend à l'opposé de la première extrémité (101, 201, 301) et la section médiane (105, 235, 305) dudit corps (130, 230, 330) ; et
ledit corps (130, 230, 330) est tubulaire et définit une lumière (132) à travers ce dernier, **caractérisé en ce que** la lumière (132) est configurée pour réduire ou pour empêcher l'écoulement de matériaux à travers cette dernière.

2. Dispositif médical implantable (100, 200, 300) selon la revendication 1, dans lequel ledit premier élément de retenue (110, 210, 310) est formé avec un matériau élastique capable de se fléchir avec des forces anatomiques dirigées radialement vers l'intérieur appliquées sur ce dernier.

3. Dispositif médical implantable (100, 200, 300) selon la revendication 2, dans lequel ledit premier élément de retenue (110, 210, 310) est compressible d'une configuration expansée initiale en réponse à l'application des forces dirigées radialement vers l'intérieur appliquées sur ce dernier, et est formé avec un matériau résilient ramenant ledit premier élément de retenue (110, 210, 310) dans la configuration expansée suite à la réduction de la force.

4. Dispositif médical implantable (100, 200, 300) selon la revendication 3, dans lequel ledit premier élément de retenue (110, 210, 310) présente au tissu, sur le site de déploiement, une surface de contact avec le tissu convexe (112, 212, 222, 312) , et est configuré et dimensionné pour résister à la migration du site de déploiement.

5. Dispositif médical implantable (100, 200, 300) selon la revendication 1, dans lequel ledit corps tubulaire (130, 230, 330) est formé avec un élément tubulaire, et au moins ledit premier élément de retenue (110, 210, 310) est formé en inversant une extrémité de l'élément tubulaire et étendant l'extrémité libre (111, 211, 311) de l'extrémité inversée vers la section médiane (105, 235, 305) de l'élément tubulaire sur une distance suffisante pour amener l'extrémité inversée de l'élément tubulaire à résister au retour à sa configuration tubulaire non inversée initiale.

6. Dispositif médical implantable (100, 200, 300) selon la revendication 5, dans lequel ledit élément tubulaire est formé à partir d'une pluralité de filaments entrelacés.

7. Dispositif médical implantable (100, 200, 300) selon la revendication 1, dans lequel ledit premier élément de retenue (110, 210, 310) est formé à partir d'un élément en forme de bol séparé dudit corps (130, 230, 330).

8. Dispositif médical implantable (100, 200, 300) selon la revendication 7, dans lequel ledit premier élément de retenue (110, 210, 310) est mobile par rapport audit corps (130, 230, 330).

9. Dispositif médical implantable (100, 200, 300) selon la revendication 7, dans lequel ledit corps (130, 230, 330) est formé à partir d'un matériau élastique permettant audit premier élément de retenue (110, 210, 310) d'être déplacé à l'opposé dudit deuxième élément de retenue (120, 220, 320).

10. Dispositif médical implantable (100, 200, 300) selon l'une quelconque des revendications 1 à 9, dans lequel la surface de contact avec le tissu (112, 212, 312) dudit premier élément de retenue (110, 210, 310) est convexe.

11. Dispositif médical implantable (100, 200, 300) selon l'une quelconque des revendications 1 à 10, dans lequel le dispositif est configuré pour être implanté par rapport à un pylore d'un patient, dans lequel :
le premier élément de retenue (110, 210, 310) est un élément de retenue gastrique ; et
le deuxième élément de retenue (120, 220, 320) est un élément de retenue duodénal ;
ledit élément de retenue gastrique a un bord libre dudit élément de retenue gastrique espacé de la première extrémité (101, 201, 301) dudit corps (130, 230, 330) dans une direction vers la section médiane (105, 235, 305) dudit corps (130, 230, 330) sur une étendue suffisante pour rester dans une telle configuration lorsque ledit dispositif médical implantable (100, 200, 300) est implanté par rapport au pylore du patient avec ledit élément de retenue gastrique positionné dans l'estomac du patient ; et
ledit élément de retenue gastrique est souple, de manière résiliente, pour se contracter d'une configuration expansée à une configuration comprimée suite à l'application de forces péristaltiques sur ce dernier à partir de l'antre de l'estomac du patient, et pour revenir à la configuration expansée après le passage d'une onde péristaltique dudit élément de retenue gastrique.

12. Dispositif médical implantable (100, 200, 300) selon la revendication 11, dans lequel ledit élément de retenue gastrique présente une surface de contact avec le tissu convexe (112, 212, 222, 312) à l'antre de l'estomac du patient.

13. Dispositif médical implantable (100, 200, 300) selon la revendication 11, dans lequel :
ledit dispositif médical implantable (100, 200, 300) est formé à partir d'un élément tubulaire ayant une première extrémité (101, 201, 301), une deuxième extrémité (103, 203, 303) et une section médiane (105, 235, 305) entre elles ; et
ledit élément de retenue gastrique est formé en inversant la première extrémité (101, 201, 301) de l'élément tubulaire et en tirant son extrémité libre (111, 211, 311) vers la deuxième extrémité (103, 203, 303) de l'élément tubulaire sur une distance suffisante pour rester dans la configuration inversée lorsqu'il est implanté par rapport à l'antre de l'estomac du patient et lorsqu'il est comprimé par une onde péristaltique de l'antre.

14. Dispositif médical implantable (100, 200, 300) selon la revendication 11, dans lequel ledit corps (130, 230, 330) et au moins ledit élément de retenue gastrique sont formés séparément et couplés ensemble pour permettre un mouvement relatif entre eux.
